# EUROPÄISCHE PATENTSCHRIFT

(11) **EP 0 636 367 B1**
(45) Veröffentlichungstag und Bekanntmachung des Hinweises auf die Patenterteilung: **29.03.2000**
(21) Anmeldenummer: 94110246.9
(22) Anmeldetag: 01.07.1994
(51) Int. Cl.: A61K 31/135

(54) **Verwendung von Phenalkylaminen zur Herstellung von cholesterinsenkenden Arzneimitteln**
Use of phenylalkylamines for producing cholesterol lowering agents
Utilisation des phénylalkylamines pour la préparation des agents pour abaisser le taux de cholestérol

(30) Priorität: 14.07.1993 CH 210793; 28.04.1994 CH 132094
(43) Veröffentlichungstag der Anmeldung: 01.02.1995
(73) Patentinhaber: F. HOFFMANN-LA ROCHE AG, 4002 Basel (CH)
(72) Erfinder: Aebi, Johannes, CH-4051 Basel (CH); Guerry, Philipe, CH-4055 Basel (CH); Jolidon, Synèse, Dr., CH-4223 Blauen (CH); Morand, Olivier, F-68220 Hegenheim (FR)
(74) Vertreter: Mahé, Jean

(56) Entgegenhaltungen:
- EP-A- 0 401 798
- EP-A- 0 410 359
- EP-A- 0 464 465
- JOURNAL OF ANTIMICROBIAL CHEMOTHERAPY, Bd.32, Nr.6, 1993 Seiten 837 - 842 GEBRE-HIWOT, A. ET AL 'THE IN-VITRO ANTI-LEISHMANIAL ACTIVITY OF INHIBITORS OF ERGOSTEROL BIOSYNTHESIS'
- PROCEEDINGS OF THE THIRD INTERNATIONAL SYMPOSIUM ON MOLECULAR ASPECTS OF CHEMOTHERAPY, Juni 1991, GDANSK, POLAND Seiten 143 - 152 JOLIDON, S. ET AL 'INHIBITORS OF 2,3-OXIDOSQUALENE-LANOSTEROL CYCLASE AS ANTIFUNGAL AGENTS'
- BIOCHEMICAL PHARMACOLOGY, Bd.37, Nr.10, 1988 Seiten 1955 - 1964 GERST, N. ET AL 'POTENT INHIBITION OF CHOLESTEROL BIOSYNTHESIS IN 3T3 FIBROBLASTS BY N-((1,5,9)-TRIMETHYLDECYL)- 4alpha,10-DIMETHYL-8-AZA-TRANS-DECAL-3beta -OL, A NEW 2,3-OXIDOSQUALENE CYCLASE INHIBITOR'

## Beschreibung

Die Erfindung betrifft die Verwendung der Verbindungen der Formel 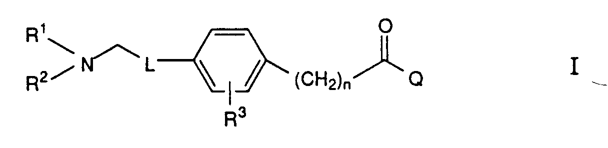 worin
- eins von R¹ und R²: C₁₋₇-Alkyl und das andere C₁₋₇Alkyl oder C₂₋₆-Alkenyl-methyl,
- L: gegebenenfalls über ein O-Atom an die Phenylgruppe gebundenes C₁₋₁₁-Alkylen oder C₂₋₁₁-Alkenylen, oder L 1,4-Phenylen,
- n: = 0 oder, falls L ein O-Atom enthält, n = 0 oder 1,
- Q: C₁₋₇-Alkyl, C₂₋₁₀-Alkenyl oder eine Gruppe der Formel Q': 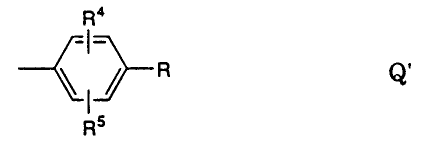
- R: H, Halogen, CF₃, CN oder NO₂,
- R³ und R⁴: H, C₁₋₄-Alkyl oder Halogen sind und
- R⁵: H oder, falls R H ist, H oder Halogen ist,
und ihrer pharmazeutisch annehmbaren Säureadditionssalze bei der Herstellung von Cholesterin senkenden Arzneimitteln.

Ferner betrifft die Erfindung neue unter die Formel I fallende Verbindungen, wie die folgenden:
4-[[6-(Allylmethylamino)hexyl]oxy]-3-chlorbenzophenon,
4-[[6-(Allylmethylamino)hexyl]oxy]-3,4'-dibrombenzophenon,
4-[[4-(Allylmethylamino)-2-butenyl]oxy]-3,4'-dibrombenzophenon,
3-Chlor-4'-jod-4-[[6-(allylmethylamino)hexyl]oxy]benzophenon,
4'-Brom-3-chlor-4-[[6-(allylmethylamino)hexyl]oxy]benzophenon,
2,4-[[(4-Dimethylamino)-2-butenyl]oxy]-3,4'-dibrombenzophenon,
4-[[4-(Dimethylamino)-2-butenyl]oxy]-3-chlorbenzophenon,
4'-Brom-3-chlor-4-[[6-(dimethylamino)hexyl]oxy]benzophenon,
3,4-Dichlorphenyl-4'-[(dimethylamino)methyl]-4-biphenylylketon,
4'-[(Allylmethylamino)methyl]-4-biphenylyl-3,4-dichlorphenylketon,
(RS)-4'-(Dimethylaminomethyl)-4-biphenyl-2,6-dimethyl-5-heptenylketon,
p-Bromphenyl-2-chlor-4'-[(dimethylamino)methyl]-4-biphenylylketon,
4'-[(Dimethylamino)methyl]-4-biphenylyl-propylketon,
[4-[6-(Allyl-methyl-amino)-hexyloxy]-phenyl]-(4-brom-phenyl)-methanon,
[4-[4-(Allyl-methyl-amino)-butoxy]-phenyl]-(4-brom-phenyl)-methanon,
[4-[6-(Allyl-methyl-amino)-hexyloxy]-3-fluor-phenyl]-(4-brom-phenyl)-methanon,
[4-[6-(Allyl-methyl-amino)-hexyloxy]-2-fluor-phenyl]-(4-brom-phenyl)-methanon,
(E)-[4-[4-(Allyl-methyl-amino)-but-2-enyloxy]-phenyl]-(4-trifluormethyl-phenyl)-methanon,
(E)-[4-[4-(Allyl-methyl-amino)-but-2-enyloxy]-3-fluor-phenyl]-(4-brom-phenyl)-methanon,
(E)-1-[4-[4-(Allyl-methyl-amino)-but-2-enyloxy]-phenyl]-5-methyl-hexan-1-on,
(E)-[4-[4-(Allyl-methyl-amino)-but-2-enyloxy]-phenyl]-(4-jod-phenyl)-methanon,
(E)-1-[4-(Allyl-methyl-amino)-but-2-enyloxy]-3-fluor-phenyl]-5-methyl-hexan-1-on,
(E)-[4-[4-(Allyl-methyl-amino)-but-2-enyloxy]-3-fluor-benzoyl]-benzonitril,
(E)-4-[4-[4-(Allyl-methyl-amino)-but-2-enyloxy]-benzoyl]-benzonitril,
(E)-[4-[4-(Allyl-methyl-amino)-but-2-enyloxy]-3-fluor-phenyl]-(2,6-difluor-phenyl)-methanon,
(E)-1-[4-[4-(Allyl-methyl-amino)-but-2-enyloxy]-phenyl]-5-methyl-hex-4-en-1-on,
(E)-[4-[4-(Allyl-methyl-amino)-but-2-enyloxy]-2-fluor-phenyl]-(4-brom-phenyl)-methanon,
(E)-[4-[4-(Allyl-methyl-amino)-but-2-enyloxy]-phenyl]-(4-fluor-phenyl)-methanon,
(E)-1-[4-[4-(Allyl-methyl-amino)-but-2-enyloxy]-3-fluor-phenyl]-6-methyl-hept-5-en-2-on,
(E)-2-[4-[4-(Allyl-methyl-amino)-but-2-enyloxy]-3-fluor-phenyl]-1-(4-brom-phenyl)-ethanon,
(E)-2-[4-[4-(Allyl-methyl-amino)-but-2-enyloxy)-phenyl]-1-(4-brom-phenyl)-ethanon,
(E)-(4-Brom-phenyl)-[4-[4-(ethyl-methyl-amino)-but-2-enyloxy]-phenyl]-methanon,
4'[(Allylmethylamino)methyl]-2-chlor-4-biphenylyl-p-bromphenylketon.

Die Ausdrücke "Alkyl" und "Alkylen" bezeichnen geradkettige oder verzweigte, gesättigte Kohlen wasserstoffreste mit einer bzw. zwei freien Valenzen, wie Methyl, Aethyl, Propyl, Isobutyl und t-Butyl, bzw. Methylen, Pentamethylen und Hexamethylen. Die Ausdrücke "Alkenyl" und "Alkenylen" bezeichnen geradkettige oder verzweigte, eine Doppelbindung enthaltende Kohlenwasserstoffreste mit einer bzw. zwei freien Valenzen, wie Vinyl und Propenyl, bzw. Propenylen.

Als pharmazeutisch annehmbare Säureadditionssalze kommen Salze der Verbindungen I mit anorganischen und organischen Säuren, wie HCl, HBr, H₂SO₄, HNO₃, Citronensäure, Essigsäure, Bernsteinsäure, Fumarsäure, Weinsäure, Methansulfonsäure und p-Toluolsulfonsäure, in Betracht.

Bevorzugte Verbindungen der Formel I sind diejenigen, worin n = 0 und R⁵ H ist.

Bevorzugte Verbindungen der Formel I sind ferner diejenigen, worin
a) R¹ Methyl und R² Methyl, Aethyl, Propyl oder Allyl und/oder
b) L die Gruppe -CH=CHCH₂O-, insbesondere in trans-Form, -(CH₂)₅ -, -(CH₂)₆-, -(CH₂)₃O-, -(CH₂)₅O-, -(CH₂)₆O- oder 1,4-Phenylen und/oder
c) R³ H, Br, Cl, F oder CH₃ und/oder
d) Q Propyl, Pentyl, Isohexyl, 4-Methyl-3-pentenyl oder 2,6-Dimethyl-5-heptenyl oder
e) Q eine Gruppe Q' ist, in der R H, Br, Cl, F, J, CF₃, CN oder NO₂ und/oder R⁴ H, Br, Cl, F, oder CH₃ und/oder R⁵ H oder F sind.

Besonders bevorzugt sind die Verbindungen der Formel I, worin
a) L C₅₋₁₁-Alkylen oder C₅₋₁₁-Alkylenoxy, insbesondere -(CH₂)₆- oder -(CH₂)₅O-; C₃₋₁₁-Alkenylenoxy, insbesondere -CH=CHCH₂O-, oder 1,4-Phenylen und/oder
b) R³ H oder Halogen und/oder
c) Q C₂₋₁₀-Alkenyl, insbesondere 4-Methyl-3-pentenyl; oder eine Gruppe Q', in der R CN, NO₂ oder Halogen, insbesondere Br, Cl oder F, und R⁴ H oder Cl ist,
speziell diejenigen, worin
a) R¹ Methyl und R² Methyl oder Allyl und/oder
b) L -(CH₂)₅O-, -CH=CHCH₂O- oder 1,4-Phenylen und/oder
c) R³ H oder F und/oder
d) Q 4-Methyl-3-pentenyl oder eine Gruppe Q', in der R Br, Cl, CN oder NO₂, R⁴ H oder Cl und R⁵ H ist.

Beispiele von bevorzugten Verbindungen sind
trans-4-[[4-(Allylmethylamino)-2-butenyl]oxy]-4'-bromobenzophenon,
trans-4-[[4-(Allylmethylamino)-2-butenyl]oxy]-4'-nitrobenzophenon,
p-[[4'-[(Allylmethylamino)methyl]-4-biphenylyl]carbonyl]benzonitril,
2-Chlor-4-nitrophenyl-4'-[(dimethylamino)methyl]-4-biphenylyl-keton,
trans-4-[[4-(Allylmethylamino)-2-butenyl]oxy]-2',4'-dichlorobenzophenon,
[4-[6-(Allyl-methyl-amino)-hexyloxy]-phenyl]-(4-brom-phenyl)-methanon,
[4-[6-(Allyl-methyl-amino)-hexyloxy]-3-fluor-phenyl]-(4-brom-phenyl)-methanon,
[4-[6-(Allyl-methyl-amino)-hexyloxy]-2-fluor-phenyl]-(4-brom-phenyl)-methanon,
(E)-[4-[4-(Allyl-methyl-amino)-but-2-enyloxy]-3-fluor-phenyl]-(4-brom-phenyl)-methanon,
(E)-1-[4-[4-(Allyl-methyl-amino)-but-2-enyloxy]-phenyl]-5-methyl-hex-4-en-1-on,
(E)-[4-[4-(Allyl-methyl-amino)-but-2-enyloxy]-2-fluor-phenyl]-(4-brom-phenyl)-methanon.

Die Verbindungen der Formel I und ihre Salze können wie in den US Patentschriften Nr. 5106878, 5137920 und 5177067 beschrieben hergestellt werden. Diejenigen Verbindungen, die in diesen Patentschriften nicht spezifisch genannt sind, sind Gegenstand der vorliegenden Erfindung. Die Herstellung solcher neuen Verbindungen ist in den nachfolgenden Beispielen beschrieben.

Die Verbindungen I und ihre Salze haben Cholesterin senkende Wirksamkeit und können daher insbesondere bei der Bekämpfung bzw. Verhütung der für die Mehrzahl der cardiovaskulären Krankheiten verantwortlichen Hypercholesterinämie und Atherosklerose verwendet werden.

Zum Beweis der Cholesterin senkenden Wirksamkeit der Verbindungen I und ihrer Salze wurde das durch D.L. Brasaemle und Attie A.D. (Biotechniques 6, 1988, 418-419) modifizierte Experiment von M. Krieger (Anal. Biochem. 135, 1983, 383-391) durchgeführt. In diesem Experiment verwendet man die Eigenschaft der Cholesterinsynthesehemmer, die Zellen CHO-K1 (Ovarzellen vom chinesischen Hamster) gegen die cytotoxischen Effekte des Polyenantibiotikums Amphotericin B zu schützen. Die Hemmung der Cholesterinsynthese wird als Schutz der lebendigen Zellen ausgedrückt und dieser Schutz wiederum als Anzahl der überlebenden Zellen im Vergleich zu unbehandelten Zellen. Die EC₅₀-Werte in nM/l in der nachfolgenden Tabelle A entsprechen der Konzentration, bei der 50% der Zellen überleben:

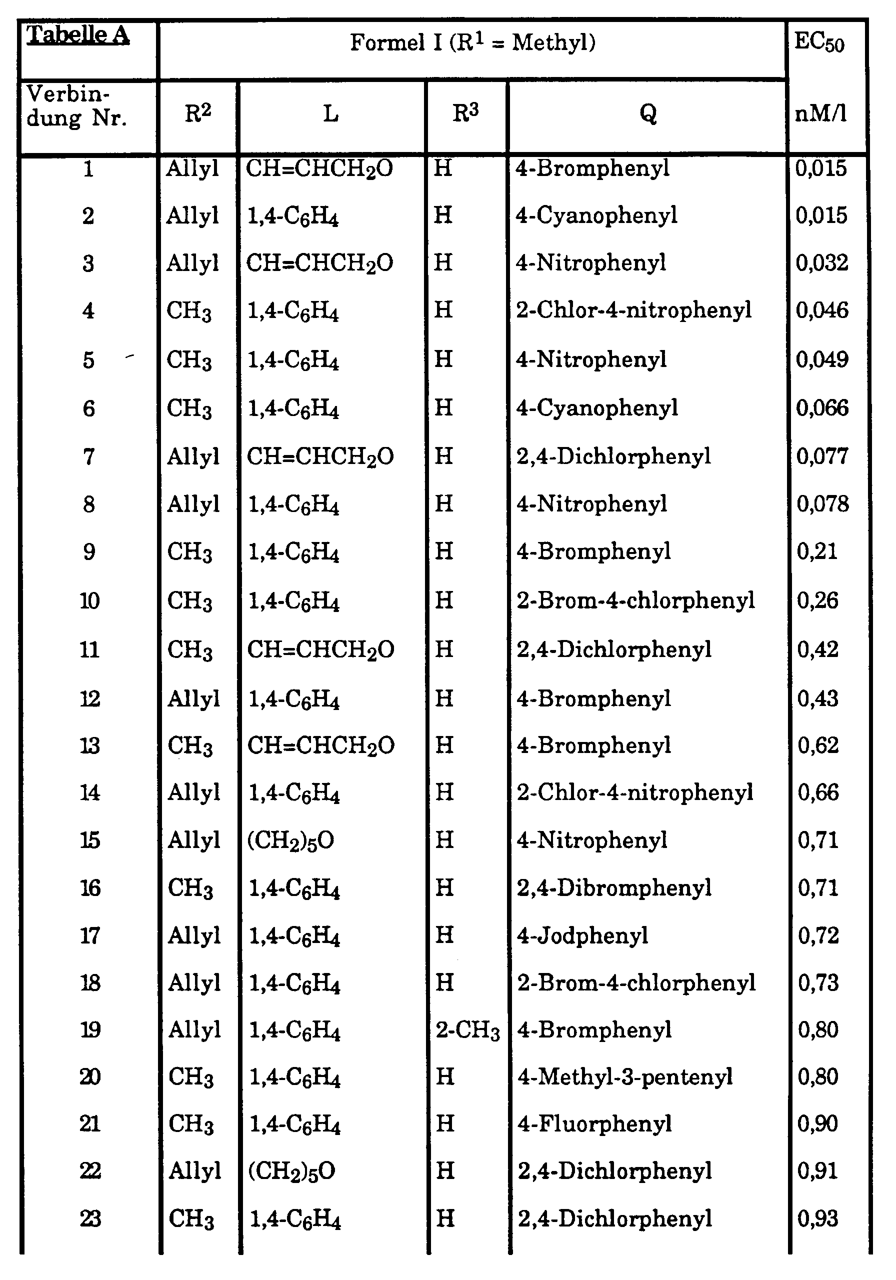

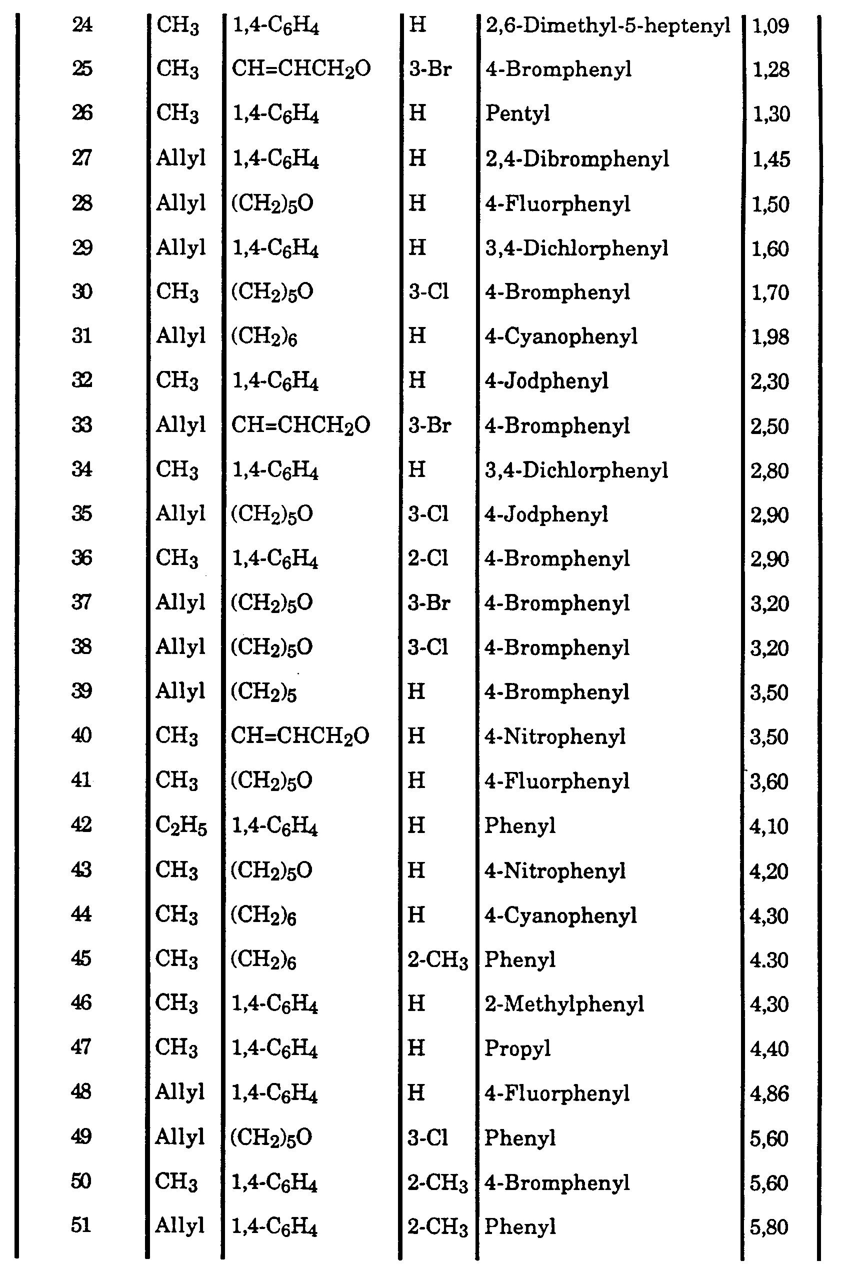

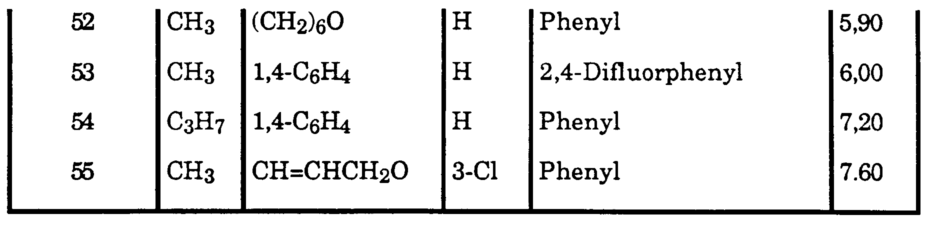

Im obigen Experiment wurde für das 2,4-Difluorphenyl-4'-[(allyl-methylamino)methyl]-4-biphenylylketon-hydrochlorid ein EC₅₀-Wert von 4,00 nM/l ermittelt.

Zum weiteren Beweis der Cholesterin senkenden Wirksamkeit der Verbindungen der Formel I und ihrer Salze wurde analog dem in J. Biol. Chem. 256 (1981), 11923-11931 beschriebenen Experiment verfahren. Dabei wurde die Hemmung der Cholesterinsynthese in menschlichen Hepatomazellen (Hep G2) anhand der parallel induzierten Aufregulierung des LDL-Rezeptors ermittelt. Die Zellen wurden in Microtiterplatten gesät und mit dem Cholesterinsynthesehemmer behandelt. Die Konzentration des LDL-Rezeptors wird mittels einer ELISA-Methodik gemessen, wobei man den C7-LDL-Antikörper als primären Antikörper verwendet. Die EC₅₀-Werte in nM/l in der nachfolgenden Tabelle B entsprechen der Konzentration des Cholesterinsynthesehemmers, bei der die Aktivität des Rezeptors im Vergleich zur Kontrolle (d.h. nicht behandelten Zellen) um 50% erhöht wird.

**Tabelle B**

| | Formel I (R¹ = Methyl) | | | | | EC₅₀ |
|---|---|---|---|---|---|---|
| Verbindung Nr. | R² | L | R³ | Q | n | nM/l |
| 1 | Allyl | CH=CHCH₂O | H | 4-Bromphenyl | 0 | 50 |
| 2 | Allyl | 1,4-C₆H₄ | H | 4-Cyanophenyl | 0 | 43 |
| 3 | Allyl | CH=CHCH₂O | H | 4-Nitrophenyl | 0 | 63 |
| 4 | CH₃ | 1,4-C₆H₄ | H | 2-Cl-4-NO₂Phenyl | 0 | 153 |
| 7 | Allyl | CH=CHCH₂O | H | 2,4-(Cl)-Phenyl | 0 | 82 |

| Beispiel Nr. | | | | | | |
|---|---|---|---|---|---|---|
| 9a | Allyl | (CH₂)₅O | H | 4-Bromphenyl | 0 | 51 |
| 9c | Allyl | (CH₂)₅O | 3-F | 4-Bromphenyl | 0 | 203 |
| 9d | Allyl | (CH₂)₅O | 2-F | 4-Bromphenyl | 0 | 57 |
| 9f | Allyl | CH=CHCH₂O | 3-F | 4-Bromphenyl | 0 | 10 |
| 9g | Allyl | CH=CHCH₂O | H | Isohexyl | 0 | 177 |
| 9h | Allyl | CH=CHCH₂O | H | 4-Jodphenyl | 0 | 94 |
| 9j | Allyl | CH=CHCH₂O | 3-F | 4-Cyanophenyl | 0 | 19 |
| 9l | Allyl | CH=CHCH₂O | 3-F | 2,6-(F)₂-Phenyl | | 220 |
| 9m | Allyl | CH=CHCH₂O | H | 4-Methyl-3-pentenyl | 0 | 34 |
| 9n | Allyl | CH=CHCH₂O | 2-F | 4-Bromphenyl | 0 | 133 |
| 9p | Allyl | CH=CHCH₂O | 3-F | 4-Methyl-3-pentenyl | 1 | 98 |
| 9r | Allyl | CH=CHCH₂O | H | 4-Bromphenyl | 1 | 222 |
| 9s | C₂H₅ | CH=CHCH₂O | H | 4-Bromphenyl | 0 | 122 |

Die 2- bzw. 3-Stellungen eines Substituenten R³ in den obigen Tabellen A und B entsprechen der ortho- bzw. meta-Stellung zu der in der Formel I enthaltenen Gruppe -(CH₂)ₙC(O)Q.

Die Toxizität dieser Verbindungen ist gering, hat doch beispielsweise die Verbindung Nr. 20 eine LD₅₀ von 1250-2500 mg/kg per os bei der Maus.

Die Verbindungen I und ihre Salze können als Wirkstoffe in pharmazeutischen Präparaten Verwendung finden. Die pharmazeutischen Präparate werden oral, z.B. in Form von Tabletten, Lacktabletten, Dragées, Hart- und Weichgelatinekapseln, Lösungen, Emulsionen oder Suspensionen, verabreicht. Zur Herstellung solcher Präparate kann man den Wirkstoff mit pharmazeutisch inerten, anorganischen oder organischen Trägern vermischen. Als Träger kann man für Tabletten, Lacktabletten, Dragées und Hartgelatinekapseln beispielsweise Lactose, Maisstärke, Talk, Stearinsäure oder deren Salze verwenden. Für Weichgelatinekapseln eignen sich als Träger beispielsweise pflanzliche Oele, Wachse oder Fette; je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Saccharose, Invertzucker und Glukose. Die pharmazeutischen Präparate können daneben noch Konversierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Wie eingangs erwähnt sind Cholesterin senkende Arzneimittel, die eine Verbindung der Formel I oder ein pharmazeutisch annehmbares Salz davon enthalten, ebenfalls Gegenstand der vorliegenden Erfindung, weiterhin auch ein Verfahren zur Herstellung solcher Arzneimittel, welches dadurch gekennzeichnet ist, dass man eine oder mehrere der genannten Wirkstoffe und gegebenenfalls einen oder mehrere andere therapeutisch wertvolle Stoffe in eine galenische Darreichungsform bringt. Wie eingangs erwähnt können diese Wirkstoffe bei der Bekämpfung bzw. Verhütung von Krankheiten, wie Hypercholesterinämie und Atherosklerose verwendet werden. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 2 mg bis etwa 2 g, vorzugsweise von etwa 10 bis etwa 100 mg angemessen sein. Dabei kann die Tagesdosis in einer, zwei oder drei Einzeldosen, z.B. zu einer oder zu den Mahlzeiten, eingenommen werden .

In den nachfolgenden Beispielen ist die Herstellung von bisher nicht bekannten Verbindungen der Formel I beschrieben.

### Beispiel 1

Zu einer Lösung von 34,5 g 1,6-Dibromhexan, 9,9 g 3-Chlor-4-hydroxybenzophenon und 1,6 g Tetrabutylammoniumbromid in 100 ml Methylenchlorid gibt man 100 ml einer 10-proz. wässrigen Natronlauge. Das heterogene Gemisch wird über Nacht bei Raumtemperatur gerührt. Die organische Phase wird abgetrennt, über Natriumsulfat getrocknet und eingedampft. Durch Chromatographieren des Rückstandes an Kieselgel mit Hexan/Essigsäureäthylester 7:3 erhält man 4-[(6-Bromhexyl)oxy]-3-chlorbenzophenon, Smp. 58°C.

Eine Lösung von 3,0 g des erhaltenen Benzophenons in 30 ml Aethanol wird mit 16 ml einer 33-proz. Lösung von N-Allyl-methylamin in Aethanol für 1,5 Stunden in einem Druckrohr auf 90°C erhitzt. Nach dem Abkühlen wird das Gemisch auf Wasser gegossen und dreimal mit Essigester extrahiert. Die über Natriumsulfat getrockneten organischen Phasen werden eingedampft, und der Rückstand wird mit Hexan/Essigester (7:3) an Aluminiumoxid neutral chromatographiert. Man erhält das 4-[[6-(Allylmethylamino)hexyl]oxy]-3-chlorbenzophenon, Smp. des Hydrochlorids 133°C.

### Beispiel 2

Analog Beispiel 1 erhält man
a) aus 3,4'-Dibrom-4-hydroxybenzophenon, via 4-[(6-Bromhexyl)oxy]-3,4'-dibrombenzophenon, Smp. 97°C, das 4-[[6-(Allylmethylamino)hexyl]oxy]-3,4'-dibrombenzophenon, Smp. des Hydrochlorids 126-127°C,
b) aus 3,4'-Dibrom-4-hydroxybenzophenon und trans 1,4-Dibrombuten, via 4-[(4-Brom-2-butenyl)oxy]-3,4'-dibrombenzophenon, das 4-[[4-(Allylmethylamino)-2-butenyl]oxy]-3,4'-dibrombenzophenon, Smp. des Hydrochlorids 115-116°C,
c) aus 3-Chlor-4'-jod-4-hydroxybenzophenon und 1,6-Dibromhexan, via 3-Chlor-4'-jod-4-[(6-bromhexyl)oxy]benzophenon, das 3-Chlor-4'-jod-4-[[6-(allylmethylamino)hexyl]oxy]benzophenon, das in das Hydrochlorid übergeführt wird, MS: m/e 511 (M⁺, 2,4%), 484 (2%), 482 (4%), 231 (2,5%), 154 (3,3%), 84 (100%),
d) via 4'-Brom-3-chlor-4-[(6-bromhexyl)oxy]benzophenon (Beispiel 3c), das 4'-Brom-3-chlor-4-[[6-(allylmethylamino)hexyl]oxy]benzophenon, das in das Hydrochlorid übergeführt wird, MS: m/e 465 (M⁺, 2%), 463 (1,5%), 436 (4%), 434 (3%), 155 (3%), 154 (4%), 84 (100%).

### Beispiel 3

Analog Beispiel 1 erhält man
a) via 4-[(4-Brom-2-butenyl)oxy]-3,4'-dibrombenzophenon (Beispiel 2b) mit Dimethylamin an Stelle von N-Allyl-methylamin, das 2,4-[[(4-Dimethylamino)-2-butenyl]oxy]-3,4'-dibrombenzophenon, das in das Hydrochlorid übergeführt wird, Smp. 166-167°C,
b) aus 3-Chlor-4-hydroxybenzophenon und 1,4-Dibrombuten, via 4-[(4-Brom-2-butenyl)oxy]-3-chlorbenzophenon, Smp. 96-97°C, das 4-[[4-(Dimethylamino)-2-butenyl]oxy]-3-chlorbenzophenon, das in das Hydrochlorid übergeführt wird, Smp. 195°C
c) aus 4'-Brom-3-chlor-4-hydroxybenzophenon und 1,6-Dibromhexan, via 4'-Brom-3-chlor-4-[(6-bromhexyl)oxy]benzophenon, das 4'-Brom-3-chlor-4-[[6-(dimethylamino)hexyl]oxy]benzophenon, das in das Hydrochlorid übergeführt wird, MS: m/e 402 (M⁺⁻Cl, 0,2%), 185 (1,3%), 183 (1,6%), 155 (2%), 128 (4%), 58 (100%).

### Beispiel 4

a) 35 ml Nitrobenzol werden in einem Eisbad abgekühlt und dann nacheinander mit 5,2 g Aluminiumchlorid und 5,0 g 4-Methylbiphenyl behandelt. Das Gemisch wird auf Raumtemperatur gebracht und dann mit 7,7 g 3,4-Dichlorbenzoylchlorid versetzt. Das Gemisch wird bei Raumtemperatur gerührt, auf Wasser gegossen und mit Methylenchlorid extrahiert. Die Extrakte werden mit 2N Salzsäure und Wasser gewaschen, über Magnesiumsulfat getrocknet und eingedampft. Der Rückstand wird an Kieselgel mit Toluol/Essigester 9:1 chromatographiert. Man erhält das 3,4-Dichlorphenyl-4'-methyl-4-biphenylylketon.
b) Ein Gemisch aus 5,0 g 3,4-Dichlorphenyl-4'-methyl-4-biphenylylketon, 2,7 g N-Bromsuccinimid und 20 mg Azaisobutyronitril in 70 ml Tetrachlorkohlenstoff wird unter Rückfluss zum Sieden erhitzt. Das ausgefallene Material wird abfiltriert, und das Filtrat wird eingedampft. Der Rückstand wird aus Toluol/Cyclohexan umkristallisiert. Man erhält das 3,4-Dichlorphenyl-4'-brommethyl-4-biphenylylketon.
c) 1,0 g 3,4-Dichlorphenyl-4'-brommethyl-4-biphenylylketon und 20 ml einer 33-proz. Lösung von Dimethylamin in Aethanol werden während 4 Stunden zum Sieden erhitzt, worauf das Gemisch eingedampft wird. Der Rückstand wird in Aether aufgenommen und mit einer ätherischen Lösung von Chlorwasserstoff behandelt. Das ausgefallene Hydrochlorid wird abfiltriert und getrocknet. Man erhält das 3,4-Dichlorphenyl-4'-[(dimethylamino)methyl]-4-biphenylylketon-hydrochlorid, Smp. 223°C.

### Beispiel 5

1,0 g 3,4-Dichlorphenyl-4'-brommethyl-4-biphenylylketon, 1,5 ml N-Allylmethylamin und 0,84 g Kaliumcarbonat in 25 ml Aethanol werden während 4 Stunden unter Rückfluss zum Sieden erhitzt. Das Gemisch wird eingedampft, und der Rückstand wird mit Aether extrahiert. Die Extrakte werden über Magnesiumsulfat getrocknet und mit einer ätherischen Lösung von Chlorwasserstoff behandelt. Das ausgefallene Hydrochlorid wird abfiltriert und getrocknet. Man erhält das 4'-[(Allylmethylamino)methyl]-4-biphenylyl-3,4-dichlorphenylketon-hydrochlorid, Smp. 160°C.

### Beispiel 6

a) Eine Lösung des aus 344 mg Magnesium und 2,27 g 1,4-Dibrombenzol in 15 ml THF hergestellten Grignard-Reagenzes wird zu einer Suspension von 2 g 4-Brom-N,N-dimethylbenzylamin und 158 mg Tetrakistriphenylphosphinpalladium in 10 ml THF getropft. Die Zugabe erfolgt dabei bei Raumtemperatur und unter einer Argonatmosphäre. Nach Beendigung der Zugabe wird das Gemisch noch 5 Stunden zum Sieden erhitzt und dann unter vermindertem Druck eingedampft. Man versetzt dann mit Aether und gesättigter Ammoniumchloridlösung und trennt die wässrige Phase ab. Diese wird mit Aether extrahiert. Die organischen Auszüge werden mit gesättigter Natriumchloridlösung gewaschen, über Magnesiumsulfat getrocknet und eingedampft. Der Rückstand wird durch Chromatographie an Kieselgel unter Eluieren mit Methylenchlorid/Methanol 9:1 gereinigt. Man erhält das 4'-Brom-N,N-dimethylbiphenylmethanamin, Smp. 60-62°C.
b) Eine Lösung des aus 0, 94 g 4'-Brom-N,N-dimethylbiphenylmethanamin und 146 mg Magnesium in 5 ml THF hergestellten Grignard-Reagenzes wird eine Lösung von 1.07 g Citronellal in 10 ml THF zugetropft. Die Zugabe erfolgt dabei bei Raumtemperatur und unter einer Argonatmosphäre. Das Gemisch wird dann 6 Stunden bei Raumtemperatur gerührt und anschliessend mit 50 ml gesättigter Ammoniumchloridlösung hydrolysiert. Man extrahiert mit Aether, trocknet die Extrakte über Magnesiumsulfat und dampft sie ein. Nach Chromatographie an Kieselgel mit Methylenchlorid/Methanol 9:1 als Elutionsmittel erhält man das (RS)-4'-[(Dimethylaminomethyl)-4-biphenylyl]-α-(2,6-dimethyl-5-heptenyl)-methanol, MS m/e: M⁺ 365 (21%), 321(19%), 280 (36%), 58 (100%).
c) Eine Lösung von 406 mg DMSO in 2 ml Methylenchlorid wird zu einer Lösung von 327 mg Oxalylchlorid in 10 ml Methylenchlorid bei -70°C gegeben. Das Reaktionsgemisch wird während 2 Minuten gerührt, worauf eine Lösung von 810 mg des Produkts von b) in 5 ml Methylenchlorid dazugegeben wird. Man rührt noch weitere 15 Minuten und versetzt das Reaktionsgemisch dann bei -70°C mit 1,18 g Triäthylamin. Man lässt das Reaktionsgemisch dann auf Raumtemperatur erwärmen und versetzt mit einer wässrigen Lösung von Natriumcarbonat. Die wässrige Phase wird mit Methylenchlorid extrahiert. Die organischen Phasen werden vereinigt, mit gesättigter Natriumchloridlösung gewaschen und über Magnesiumsulfat getrocknet. Dem nach Filtrieren und Eindampfen erhaltenen Material gibt man eine heisse Lösung von 263 mg Fumarsäure in 5 ml Aethanol zu. Das ausgefallene Fumarat wird aus Aethanol umkristallisiert. Man erhält das (RS)-4'-(Dimethylaminomethyl)-4-biphenyl-2,6-dimethyl-5-heptenylketonfumarat, Smp. 116-123°C.

### Beispiel 7

a) Analog Beispiel 6 a) erhält man aus 4-Bromtoluol und 3-Chlorbrombenzol das 3-Chlor-4'-methylbiphenyl, Sdp. 110-115°C/20Pa.
b) Ein Gemisch von 4,76 g 3-Chlor-4'-methylbiphenyl, 2,94 g Hexamethylentetramin und 30 ml Trifluoressigsäure wird 5 Tage unter Rückfluss zum Sieden erhitzt. Das Reaktionsgemisch wird dann konzentriert und mit Eiswasser versetzt, worauf man während 15 Minuten rührt, mit Natriumcarbonat basisch stellt und mit Aether extrahiert. Nach eindampften der ätherischen Extrakte und Chromatographie des Rückstandes an Kieselgel mit Methylenchlorid/Methanol 9:1 als Elutionsmittel erhält man das 2-Chlor-4-(4'-methylphenyl)benzaldehyd, Sdp. 210-215°C/25Pa.
c) Analog Beispiel 6 b) und 6 c) erhält man aus 2-Chlor-4-(4'-methylphenyl)benzaldehyd und 1,4-Dibrombenzol das p-Bromphenyl-2-chlor-4'-methyl-4-biphenylketon als farbloses Oel, MS m/e : 386 (M⁺, 46%), 306 (9%), 229 (100%).
d) Analog Beispiel 4 b) erhält man aus p-Bromphenyl-2-chlor-4'-methyl-4-biphenylketon das 4'-Brommethyl-2-chlor-p-bromphenyl-4-biphenylketon.
e) Analog Beispiel 4 c) erhält man durch Behandlung des 4'-Brommethyl-2-chlor-p-bromphenyl-4-biphenylketons mit Dimethylamin und dann mit Chlorwasserstoff das p-Bromphenyl-2-chlor-4'-[(dimethylamino)methyl]-4-biphenylylketon-hydrochlorid, Smp- 189-191°C.

### Beispiel 8

Eine Lösung des aus 228 mg Magnesium und 1,42 g n-Propylbromid in 10 ml THF hergestellten Grignardreagenzes wird einer Lösung von 1,16 g 4'-[(Dimethylamino)methyl]-N-methoxy-N-methyl-4-biphenylcarboxamid in 10 ml THF bei 0°C unter Argon zugetropft. Nach Beendigung der Zugabe wird das Gemisch noch 5 Stunden bei Raumtemperatur gerührt und dann unter vermindertem Druck eingedampft. Man versetzt mit Methylenchlorid und gesättigter Ammoniumchloridlösung und trennt die wässrige Phase ab. Diese wird mit Methylenchlorid extrahiert. Die organischen Auszüge werden mit gesättigter Natriumchloridlösung gewaschen, über Magnesiumsulfat getrocknet und eingedampft. Der Rückstand wird durch Chromatographie and Kieselgel unter Eluieren mit Methylenchlorid-Methanol 95:5 gereinigt. Nach Umsetzung mit Fumarsäure in Aethanol erhält man das 4'-[(Dimethylamino)methyl]-4-biphenylyl-propylketon-fumarat, Smp. von 155-156°C.

### Beispiel 9

### Ausgangsmaterialien

A) Ein Gemisch von 41 g 4-Hydroxybenzoesäure und 400 ml Hexamethyldisilazan wird 2 Stunden am Rückfluss erhitzt, dann abgekühlt, eingeengt und in 400 ml Methylenchlorid gelöst. Nach Zugabe von 3 Tropfen DMF werden 28 ml Oxalylchlorid zugetropft. Es wird gerührt, dann eingeengt und getrocknet. Das erhaltene Säurechlorid wird mit 31 g N,O-Dimethylhydroxylamin-hydrochlorid in 520 ml Methylenchlorid suspendiert und bei 0°C während 2 Stunden mit 73 ml N-Methylmorpholin versetzt. Ueber Nacht wird aufgewärmt, in Essigester aufgenommen und mit Wasser, 10% wässrige KHSO₄- und gesättigte wässrige NaHCO₃-Lösung gewaschen. Die organische Phase wird getrocknet, abfiltriert und eingedampft. Man erhält 76 g N-Methoxy-N-methyl-trimethylsilanyloxy-benzamid, MS: m/e 238 (M⁺-CH₃).
B) Analog Absatz A) erhält man
   Ba) aus 4-Hydroxyphenylessigsäure das N-Methoxy-N-methyl-2-(4-trimethylsilanyloxy-phenyl)-acetamid, MS: m/e 267 (M⁺), 252 (M⁺-CH₃),
   Bb) aus 3-Fluor-4-hydroxy-phenylessigsäure das N-Methoxy-N-methyl-2-(3-fluor-4-trimethylsilanyloxy-phenyl)-acetamid, MS: m/e 285 (M⁺).
C) Zu einem aus 1 g Magnesium und 5,7 g 1-Brom-4-methyl-3-penten hergestellten Grignard-Reagenz wird bei 0°C eine Lösung von 6,3 g N-Methoxy-N-methyl-trimethylsilanyloxy-benzamid zugetropft. Die Reaktion wird über Nacht bei Raumtemperatur unter Rühren stehen gelassen. Es wird mit 10% wässriger KHSO₄-Lösung versetzt und dann mit Essigester extrahiert. Die organische Phase wird mit 10% wässriger NaCl-Lösung neutral gewaschen, dann getrocknet und eingeengt. Die Silylgruppe wird in 10% wässrigem THF mit 1N Salzsäure abgespalten. Dann wird in Methylenchlorid aufgenommen, getrocknet und eingedampft. Nach Chromatographie über Silicagel unter Eluieren mit Methylenchlorid/0,5% Methanol werden 2,1 g 1-(4-Hydroxy-phenyl)-5-methyl-hex-4-en-1-on erhalten. MS: m/e 204 (M⁺).
D) Analog Absatz C) erhält man aus N-Methoxy-N-methyl-2-(3-fluor-4-trimethylsilanyloxy-phenyl)-acetamid (Absatz Bb) das 1-(3-Fluor-4-hydroxy-phenyl)-6-methyl-hept-5-en-2-on, MS: m/e 236 (M⁺).
E) Eine Lösung von 45 ml n-Butyllithium (1,6M in Hexan) wird zu einer auf -78°C gekühlten Suspension von 18,2 g 1,4-Dibrombenzol in 140 ml THF getropft. Dann werden bei -78°C 10 g N-Methoxy-N-methyl-2-(3-fluor-4-trimethylsilanyloxy-phenyl)-acetamid (Absatz Bb)) in 35 ml THF zugetropft. Das Reaktionsgemisch wird 2 Stunden bei -78°C gerührt, dann 1 Stunde bei Raumtemperatur unter Rühren stehen gelassen. Nach Verdünnen mit Essigester wird mit 10% wässriger KHSO₄-, gesättigter NaHCO₃- und 10% wässriger NaCl-Lösung gewaschen. Nach Extraktion mit Essigester werden die organischen Phasen getrocknet und eingeengt. Dann wird mit 105 ml THF, 11 ml H₂O und 5 Tropfen 1N HCl die Silylgruppe abgespalten. Einengen, Auflösung in Methylenchlorid, Trocknen und Säulenchromatographie über Kieselgel mit Methylenchlorid/0,5% Methanol als Eluent ergeben 9,2 g 1-(4-Brom-phenyl)-2-(3-fluor-4-hydroxy-phenyl)-ethanon. MS: m/e 308 (M⁺, 1 Br).
F) Analog Absatz E) erhält man aus N-Methoxy-N-methyl-2-(4-trimethylsilanyloxy-phenyl)-acetamid (Absatz Ba)) das 1-(4-Brom-phenyl)-2-(4-hydroxy-phenyl)-ethanon, MS: m/e 290 (M⁺, 1 Br).
G) 14 ml Nitrobenzol werden im Eisbad gekühlt und dann nacheinander mit 3,8 g AlCl₃ und 3,7 g 5-Methyl-hexansäurechlorid in 5 ml Nitrobenzol gemischt. Das Gemisch wird gerührt und dann mit 2,7 ml 2-Fluor-anisol versetzt. Die Lösung wird über Nacht gerührt, dann auf Eiswasser gegossen und mit Methylenchlorid extrahiert. Die Extrakte werden mit Wasser und 10% wässriger NaCl-Lösung gewaschen, dann getrocknet und eingeengt und mit Pentan auskristallisiert. Es werden 5,3 g 1-(3-Fluor-4-methoxy-phenyl)-5-methyl-hexan-1-on erhalten, MS: m/e 238 (M⁺).
H) Analog Absatz G) erhält man:
   Ha) aus 4-Brom-benzoylchlorid und 2-Fluor-anisol das (4-Brom-phenyl)-(3-fluor-4-methoxy-phenyl)-methanon, Smp. 142-143°C,
   Hb) aus 4-Cyano-benzoylchlorid und 2-Fluor-anisol das 4-(3-Fluor-4-methoxy-benzoyl)-benzonitril, Smp. 132,5-133°C,
   Hc) aus 4-Brom-benzoylchlorid und 3-Fluor-anisol das (4-Brom-phenyl)-(2-fluor-4-methoxy-phenyl)-methanon, MS: m/e 308 (M⁺, 1 Br),
   Hd) aus 2,6-Difluor-benzoylchlorid und 2-Fluor-anisol das (2,6-Difluor-phenyl)-(3-fluor-4-methoxy-phenyl)-methanon, Smp. 79-83°C.
I) Eine Lösung von 3,9 g (2,6-Difluor-phenyl)-(3-fluor-4-methoxy-phenyl)-methanon (Absatz Hd)) in 30 ml Essigsäure wird mit 20 ml wässrige 62% HBr-Lösung bei 125°C gerührt, dann eingedampft, mit Toluol nachgedampft und in Essigester aufgenommen. Die organische Phase wird mit ges. wässriger NaHCO₃-Lösung und 10% NaCl-Lösung gewaschen und dann getrocknet. Es werden 3,6 g (2,6-Difluor-phenyl)-(3-fluor-4-hydroxy-phenyl)-methanon erhalten, MS: m/e 252 (M⁺).
J) Analog erhält man:
   Ja) aus (4-Brom-phenyl)-(3-fluor-4-methoxy-phenyl)-methanon (Absatz Ha)) das (4-Brom-phenyl)-(3-fluor-4-hydroxy-phenyl)-methanon, Smp. 183-184°C,
   Jb) aus (4-Brom-phenyl)-(2-fluor-4-methoxy-phenyl)-methanon (Absatz Hc) das (4-Brom-phenyl)-(2-fluor-4-hydroxy-phenyl)-methanon, MS: m/e 294 (M⁺, 1 Br),
   Jc) aus Anisol und 5-Methyl-hexansäurechlorid via 1-(4-Methoxy-phenyl)-5-methyl-hexan-1-on direkt das 1-(4-Hydroxy-phenyl)-5-methyl-hexan-1-on, MS: m/e 206 (M⁺).
K) Eine Lösung von 50 g 4-(3-Fluor-4-methoxy-benzoyl)-benzonitril in 550 ml Methylenchlorid wird bei 5°C mit 70 ml BBr₃ versetzt und bei Raumtemperatur gerührt. Unter Eiskühlung wird 1 l 1M NaOH zugetropft. Dann wird mit gesättiger wässriger NH₄Cl-Lösung und Methylenchlorid extrahiert. Die organische Phase wird mit Wasser gewaschen und getrocknet. Nach Umkristallisation aus Ether werden 34 g 4-(3-Fluor-4-hydroxy-benzoyl)-benzonitril erhalten, Smp. 168,5-169,5°C.

### Produkte

Analog Beispiel 1 erhält man
a) aus 4'-Brom-4-hydroxybenzophenon und 1,6-Dibromhexan, via 4'-Brom-4-[(6-bromhexyl)oxy]benzophenon und Reaktion mit N-Allyl-methylamin das [4-[6-(Allyl-methyl-amino)-hexyloxy]-phenyl]-(4-brom-phenyl)-methanon-hydrobromid, Smp. 117-119°C,
b) aus 4'-Brom-4-hydroxybenzophenon und 1,4-Dibrombutan, via 4'-Brom-4-[(6-brombutyl)oxy]benzophenon und Reaktion mit N-Allyl-methylamin das [4-[4-(Allyl-methyl-amino)-butoxy]-phenyl]-(4-brom-phenyl)-methanon-hydrobromid, Smp. 149-151°C,
c) aus (4-Brom-phenyl)-(3-fluor-4-hydroxy-phenyl)-methanon (Absatz Ja) und 1,6-Dibromhexan, via [4-(6-Brom-hexyl)-3-fluor-phenyl]-(4-brom-phenyl)-methanon und Reaktion mit N-Allyl-methylamin das [4-[6-(Allyl-methyl-amino)-hexyloxy]-3-fluor-phenyl]-(4-brom-phenyl)-methanon, das in das Hydrochlorid übergeführt wird, MS: m/e 447 (M⁺, 1 Br),
d) aus (4-Brom-phenyl)-(2-fluor-4-hydroxy-phenyl)-methanon (Absatz Jb) und 1,6-Dibromhexan, via [4-(6-Brom-hexyl)-2-fluor-phenyl]-(4-brom-phenyl)-methanon und Reaktion mit N-Allyl-methylamin das [4-[6-(Allyl-methyl-amino)-hexyloxy]-2-fluor-phenyl]-(4-brom-phenyl)-methanon, das in das Hydrochlorid übergeführt wird, Smp. 106-109°C,
e) aus 4'-Trifluormethyl-4-hydroxybenzophenon und (E)-1,4-Dibrombuten, via (E)-[4-[4-Brom-but-2-enyloxy]-phenyl]-(4-trifluormethyl-phenyl)-methanon und Reaktion mit N-Allyl-methylamin das (E)-[4-[4-(Allyl-methyl-amino)-but-2-enyloxy]-phenyl]-(4-trifluormethyl-phenyl)-methanon, das ins Hydrochlorid übergeführt wird, MS: m/e M 390 (M+H⁺),
f) aus (4-Brom-phenyl)-(3-fluor-4-hydroxy-phenyl)-methanon (Absatz Ja) und (E)-1,4-Dibrombuten, via (E)-[4-(4-Brom-but-2-enyloxy)-3-fluor-phenyl]-(4-brom-phenyl)-methanon und Reaktion mit N-Allyl-methylamin das (E)-[4-[4-(Allyl-methyl-amino)-but-2-enyloxy]-3-fluor-phenyl]-(4-brom-phenyl)-methanon, das ins Hydrochlorid überführt wird, MS: m/e 418 (M+H⁺, 1 Br),
g) aus 1-(4-Hydroxy-phenyl)-5-methyl-hexan-1-on (Absatz Jc) und (E)-1,4-Dibrombuten, via (E)-1-[4-[4-Brombut-2-enyloxy]-phenyl]-5-methyl-hexan-1-on und Reaktion mit N-Allyl-methylamin das (E)-1-[4-[4-(Allyl-methyl-amino)-but-2-enyloxy]-phenyl]-5-methyl-hexan-1-on, das ins Hydrochlorid überführt wird, Smp. 105-106°C,
h) aus (4-Hydroxy-phenyl)-(4-jod-phenyl)-methanon und (E)-1,4-Dibrombuten, via (E)-[4-[4-Brom-but-2-enyloxy]-phenyl]-(4-jod-phenyl)-methanon und Reaktion mit N-Allyl-methylamin das (E)-[4-[4-(Allyl-methyl-amino)-but-2-enyloxy]-phenyl]-(4-jod-phenyl)-methanon, das ins Hydrochlorid überführt wird, Smp. 152-153°C,
i) aus 1-(3-Fluor-4-methoxy-phenyl)-5-methyl-hexan-1-on (Absatz G), via 1-(3-Fluor-4-hydroxy-phenyl)-5-methyl-hexan-1-on und (E)-1-[4-Brom-but-2-enyloxy]-3-fluor-phenyl]-5-methyl-hexan-1-on und Reaktion mit N-Allyl-methylamin das (E)-1-[4-(Allyl-methyl-amino)-but-2-enyloxy]-3-fluor-phenyl]-5-methyl-hexan-1-on, das als Hydrobromid isoliert wird, Smp. 106-107°C,
j) aus 4-(3-Fluor-4-hydroxy-benzoyl)-benzonitril (Absatz K) mit (E)-1,4-Dibrombuten, via (E)-4-[4-(4-Brom-but-2-enyloxy)-3-fluor-benzoyl]-benzonitril und Reaktion mit N-Allyl-methylamin das (E)-[4-[4-(Allyl-methyl-amino)-but-2-enyloxy]-3-fluor-benzoyl]-benzonitril, MS: m/e 364 (M⁺),
k) aus 4-(4-Hydroxy-benzoyl)-benzonitril mit (E)-1,4-Dibrombuten, via (E)-4-[4-(4-Brom-but-2-enyloxy)-benzoyl]-benzonitril und Reaktion mit N-Allyl-methylamin das (E)-4-[4-[4-(Allyl-methyl-amino)-but-2-enyloxy]-benzoyl]-benzonitril, MS: m/e 346 (M⁺),
l) aus (2,6-Difluor-phenyl)-(3-fluor-4-hydroxy-phenyl)-methanon (Absatz I) und (E)-1,4-Dibrombuten, via (E)-[4-[4-Brom-but-2-enyloxy]-3-fluor-phenyl]-(2,6-difluor-phenyl)-methanon und Reaktion mit N-Allyl-methylamin das (E)-[4-[4-(Allyl-methyl-amino)-but-2-enyloxy]-3-fluor-phenyl]-(2,6-difluor-phenyl)-methanon, das als Hydrobromid isoliert wird, Smp. 162°C,
m) aus 1-(4-Hydroxy-phenyl)-5-methyl-hex-4-en-1-on (Absatz C) und (E)-1,4-Dibrombuten, via (E)-1-[4-[4-Brom-but-2-enyloxy]-phenyl]-5-methyl-hex-4-en-1-on und Reaktion mit N-Allyl-methylamin das (E)-1-[4-[4-(Allyl-methyl-amino)-but-2-enyloxy]-phenyl]-5-methyl-hex-4-en-1-on, das als Fumarat isoliert wird, MS: m/e 327 (M⁺),
n) aus (4-Brom-phenyl)-(2-fluor-4-hydroxy-phenyl)-methanon (Absatz Jb) und (E)-1,4-Dibrombuten, via (E)-[4-[4-Brom-but-2-enyloxy]-2-fluor-phenyl]-(4-brom-phenyl)-methanon und Reaktion mit N-Allyl-methyl-amin das (E)-[4-[4-(Allyl-methyl-amino)-but-2-enyloxy]-2-fluor-phenyl]-(4-brom-phenyl)-methanon, das als Hydrochlorid isoliert wird, Smp. 88-92°C,
o) aus 4-Fluor-4'-hydroxy-benzophenon und (E)-1,4-Dibrombuten, via (E)-[4-[4-Brom-but-2-enyloxy]-phenyl]-(4-fluor-phenyl)-methanon und Reaktion mit N-Allyl-methylamin das (E)-[4-[4-(Allyl-methyl-amino)-but-2-enyloxy]-phenyl]-(4-fluor-phenyl)-methanon, das als Hydrochlorid isoliert wird, MS: m/e 338 (M-H⁺),
p) aus 1-(3-Fluor-4-hydroxy-phenyl)-6-methyl-hept-5-en-2-on (Absatz D) und (E)-1,4-Dibrombuten, via (E)-1-[4-[4-Brom-but-2-enyloxy]-3-fluor-phenyl]-6-methyl-hept-5-en-2-on und Reaktion mit N-Allyl-methylamin das (E)-1-[4-[4-(Allyl-methyl-amino)-but-2-enyloxy]-3-fluor-phenyl]-6-methyl-hept-5-en-2-on, das als Fumarat isoliert wird, MS: m/e 359 (M⁺),
q) aus 1-(4-Brom-phenyl)-2-(3-fluor-4-hydroxy-phenyl)-ethanon (Absatz E) und (E)-1,4-Dibrombuten, via (E)-2-[4-[4-Brom-but-2-enyloxy]-3-fluor-phenyl]-(4-brom-phenyl)-ethanon und Reaktion mit N-Allyl-methylamin das (E)-2-[4-[4-(Allyl-methyl-amino)-but-2-enyloxy]-3-fluor-phenyl]-(4-brom-phenyl)-ethanon, das als Hydrochlorid isoliert wird, Smp. 114-116°C,
r) aus 1-(4-Brom-phenyl)-2-(4-hydroxy-phenyl)-ethanon (Absatz Fa) und (E)-1,4-Dibrombuten, via (E)-2-[4-(4-Brom-but-2-enyloxy)-phenyl]-1-(4-brom-phenyl)-ethanon und Reaktion mit N-Allyl-methylamin das (E)-2-[4-[4-(Allyl-methyl-amino)-but-2-enyloxy)-phenyl]-1-(4-brom-phenyl)-ethanon, das als Hydrochlorid isoliert wird, Smp. 150-153°C,
s) aus 4'-Brom-4-hydroxybenzophenon und (E)-1,4-Dibrombuten, via (E)-[4-(4-Brom-but-2-enyloxy)-phenyl]-(4-brom-phenyl)-methanon und Reaktion mit N-Ethyl-methylamin das (E)-(4-Brom-phenyl)-[4-[4-(ethyl-methyl-amino)-but-2-enyloxy]-phenyl]-methanon-hydrobromid, Smp. 171,5°C (Zersetzung).

### Beispiel 10

Analog Beispiel 4c) erhält man durch Behandlung des 4'-Brom-methyl-2-chlor-p-bromphenyl-4-biphenylketons mit N-Allyl-methylamin das 4'-[(Allylmethylamino)methyl]-2-chlor-4-biphenylyl-p-bromphenylketon, MS: m/e 453 (M⁺, 1 Br).

### Galenisches Beispiel.

Eine Hartgelatinekapsel enthält z.B. 3,125, 6.25, 12,5, 25 oder 50 mg einer Verbindung der Formel I oder eines Salzes davon und feinkristalline Lactose bis zu einem Gasamtfüllgewicht von 580-590 mg.

## Patentansprüche

1. Verwendung der Verbindungen der Formel 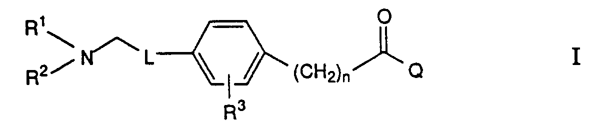 worin
eins von R¹ und R² C₁₋₇-Alkyl und das andere C₁₋₇Alkyl oder C₂₋₆-Alkenyl-methyl,
L gegebenenfalls über ein O-Atom an die Phenylgruppe gebundenes C₁₋₁₁-Alkylen oder C₂₋₁₁-Alkenylen, oder L 1,4-Phenylen,
n = 0 oder, falls L ein O-Atom enthält, n = 0 oder 1,
Q C₁₋₇-Alkyl, C₂₋₁₀-Alkenyl oder eine Gruppe der Formel Q': 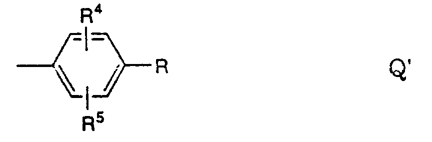
R H, Halogen, CF₃, CN oder NO₂,
R³ und R⁴ H, C₁₋₄-Alkyl oder Halogen sind und
R⁵ H oder, falls R H ist, H oder Halogen ist,
und ihrer pharmazeutisch annehmbaren Säureadditionssalze bei der Herstellung von Cholesterin senkenden Arzneimitteln.

2. Verwendung gemäss Anspruch 1, wobei n = 0 und R⁵ H ist.

3. Verwendung gemäss Anspruch 1 oder 2, wobei
a) R¹ Methyl und R² Methyl, Aethyl, Propyl oder Allyl und/oder
b) L die Gruppe -CH=CHCH₂O-, insbesondere in trans-Form, -(CH₂)₅-, -(CH₂)₆-, -(CH₂)₃O-, -(CH₂)₅O-, -(CH₂)₆O- oder 1,4-Phenylen und/oder
c) R³ H, Br, Cl, F oder CH₃ und/oder
d) Q Propyl, Pentyl, Isohexyl, 4-Methyl-3-pentenyl oder 2,6-Dimethyl-5-heptenyl oder
e) Q eine Gruppe Q' ist, in der R H, Br, Cl, F, J, CF₃, CN oder NO₂ und/oder R⁴ H, Br, Cl, F, oder CH₃ und/oder R⁵ H oder F sind.

4. Verwendung gemäss Anspruch 1 oder 2, wobei
a) L C₅₋₁₁-Alkylen oder C₅₋₁₁-Alkylenoxy, insbesondere -(CH₂)₆- oder -(CH₂)₅O-; C₃₋₁₁-Alkenylenoxy, insbesondere -CH=CHCH₂O-, oder 1,4-Phenylen und/oder
b) R³ H oder Halogen und/oder
c) Q C₂₋₁₀-Alkenyl, insbesondere 4-Methyl-3-pentenyl; oder eine Gruppe Q', in der R CN, NO₂ oder Halogen, insbesondere Br, Cl oder F, und R⁴ H oder Cl ist.

5. Verwendung gemäss Anspruch 4, wobei
a) R¹ Methyl und R² Methyl oder Allyl und/oder
b) L -(CH₂)₅O-, -CH=CHCH₂O- oder 1,4-Phenylen und/oder
c) R³ H oder F und/oder
d) Q 4-Methyl-3-pentenyl oder eine Gruppe Q', in der R Br, Cl, CN oder NO₂, R⁴ H oder Cl und R⁵ H ist.

6. Verwendung gemäss Anspruch 1 oder 2 wobei die Verbindung aus der folgenden Gruppe ausgewählt wird:
trans-4-[[4-(Allylmethylamino)-2-butenyl]oxy]-4'-bromobenzophenon
trans-4-[[4-(Allylmethylamino)-2-butenyl]oxy]-4'-nitrobenzophenon
p-[[4'-[(Allylmethylamino)methyl]-4-biphenylyl]carbonyl]benzonitril
2-Chlor-4-nitrophenyl-4'-[(dimethylamino)methyl]-4-biphenylyl-keton.
trans-4-[[4-(Allylmethylamino)-2-butenyl]oxy]-2',4'-dichlorobenzophenon.

7. Verwendung gemäss Anspruch 1, wobei die Verbindung aus der folgenden Gruppe ausgewählt wird:
[4-[6-(Allyl-methyl-amino)-hexyloxy]-phenyl]-(4-brom-phenyl)-methanon,
[4-[6-(Allyl-methyl-amino)-hexyloxy]-3-fluor-phenyl]-(4-brom-phenyl)-methanon,
[4-[6-(Allyl-methyl-amino)-hexyloxy]-2-fluor-phenyl]-(4-brom-phenyl)-methanon,
(E)-[4-[4-(Allyl-methyl-amino)-but-2-enyloxy]-3-fluor-phenyl]-(4-brom-phenyl)-methanon,
(E)-1-[4-[4-(Allyl-methyl-amino)-but-2-enyloxy]-phenyl]-5-methyl-hex-4-en-1-on,
(E)-[4-[4-(Allyl-methyl-amino)-but-2-enyloxy]-2-fluor-phenyl]-(4-brom-phenyl)-methanon.

8. Die Verbindungen der Formel I gemäss Anspruch 1 oder 2 gewählt aus der folgenden Gruppe:
4-[[6-(Allylmethylamino)hexyl]oxy]-3-chlorbenzophenon,
4-[[6-(Allylmethylamino)hexyl]oxy]3,4'-dibrombenzophenon,
4-[[4-(Allylmethylamino)-2-butenyl]oxy]-3,4'-dibrombenzophenon,
3-Chlor-4'-jod-4-[[6-(allylmethylamino)hexyl]oxy]benzophenon,
4'-Brom-3-chlor-4-[[6-(allylmethylamino)hexyl]oxy]benzophenon,
2,4-[[(4-Dimethylamino)-2-butenyl]oxy]-3,4'-dibrombenzophenon,
4-[[4-(Dimethylamino)-2-butenyl]oxy]-3-chlorbenzophenon,
4'-Brom-3-chlor-4-[[6-(dimethylamino)hexyl]oxy]benzophenon,
3,4-Dichlorphenyl-4'-[(dimethylamino)methyl]-4-biphenylylketon,
4'-[(Allylmethylamino)methyl]-4-biphenylyl-3,4-dichlorphenylketon,
(RS)-4'-(Dimethylaminomethyl)-4-biphenyl-2,6-dimethyl-5-heptenyl-keton,
p-Bromphenyl-2-chlor-4'-[(dimethylamino)methyl]-4-biphenylylketon,
4'-[(Dimethylamino)methyl]-4-biphenylyl-propylketon.

9. Die Verbindungen der Formel I gemäss Anspruch 1 gewählt aus der folgenden Gruppe:
[4-[6-(Allyl-methyl-amino)-hexyloxy]-phenyl]-(4-brom-phenyl)-methanon,
[4-[4-(Allyl-methyl-amino)-butoxy]-phenyl]-(4-brom-phenyl)-methanon,
[4-[6-(Allyl-methyl-amino)-hexyloxy]-3-fluor-phenyl]-(4-brom-phenyl)-methanon,
[4-[6-(Allyl-methyl-amino)-hexyloxy]-2-fluor-phenyl]-(4-brom-phenyl)-methanon,
(E)-[4-[4-(Allyl-methyl-amino)-but-2-enyloxy]-phenyl]-(4-trifluormethyl-phenyl)-methanon,
(E)-[4-[4-(Allyl-methyl-amino)-but-2-enyloxy]-3-fluor-phenyl]-(4-brom-phenyl)-methanon,
(E)-1-[4-[4-(Allyl-methyl-amino)-but-2-enyloxy]-phenyl]-5-methyl-hexan-1-on,
(E)-[4-[4-(Allyl-methyl-amino)-but-2-enyloxy]-phenyl]-(4-jod-phenyl)-methanon,
(E)-1-[4-(Allyl-methyl-amino)-but-2-enyloxy]-3-fluor-phenyl]-5-methyl-hexan-1-on,
(E)-[4-[4-(Allyl-methyl-amino)-but-2-enyloxy]-3-fluor-benzoyl]-benzonitril,
(E)-4-[4-[4-(Allyl-methyl-amino)-but-2-enyloxy]-benzoyl]-benzonitril,
(E)-[4-[4-(Allyl-methyl-amino)-but-2-enyloxy]-3-fluor-phenyl]-(2,6-difluor-phenyl)-methanon,
(E)-1-[4-[4-(Allyl-methyl-amino)-but-2-enyloxy]-phenyl]-5-methyl-hex-4-en-1-on,
(E)-[4-[4-(Allyl-methyl-amino)-but-2-enyloxy]-2-fluor-phenyl]-(4-brom-phenyl)-methanon,
(E)-[4-[4-(Allyl-methyl-amino)-but-2-enyloxy]-phenyl]-(4-fluor-phenyl)-methanon,
(E)-1-[4-[4-(Allyl-methyl-amino)-but-2-enyloxy]-3-fluor-phenyl]-6-methyl-hept-5-en-2-on,
(E)-2-[4-[4-(Allyl-methyl-amino)-but-2-enyloxy]-3-fluor-phenyl]-1-(4-brom-phenyl)-ethanon,
(E)-2-[4-[4-(Allyl-methyl-amino)-but-2-enyloxy)-phenyl]-1-(4-brom-phenyl)-ethanon,
(E)-(4-Brom-phenyl)-[4-[4-(ethyl-methyl-amino)-but-2-enyloxy]-phenyl]-methanon,
4'-[(Allylmethylamino)methyl]-2-chlor-4-biphenylyl-p-bromphenylketon.

## Claims

1. Use of the compounds of the formula 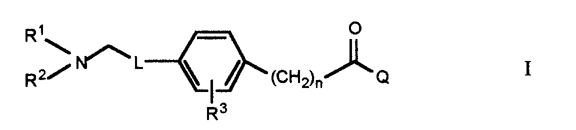 wherein
one of R¹ and R² is C₁₋₇-alkyl and the other is C₁₋₇-alkyl or C₂₋₆-alkenyl-methyl,
L is C₁₋₁₁-alkylene or C₂₋₁₁-alkenylene optionally bonded to the phenyl group via an O atom or L is 1,4-phenylene,
n = 0 or, where L contains an O atom, n = 0 or 1;
Q is C₁₋₇-alkyl, C₂₋₁₀-alkenyl or a group of formula Q': 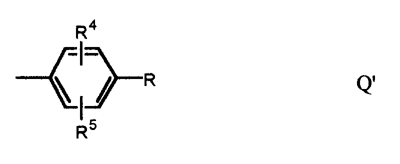
R is H, halogen, CF₃, CN or NO_{2,}
R³ and R⁴ are H, C₁₋₄-alkyl or halogen and
R⁵ is H or, where R is H, H or halogen,
and their pharmaceutically acceptable acid addition salts for the manufacture of cholesterol-lowering medicaments.

2. Use in accordance with claim 1, whereby n = 0 and R⁵ is H.

3. Use in accordance with claim 1 or 2, whereby
a) R¹ is methyl and R² is methyl, ethyl, propyl or allyl and/or
b) L is the group -CH=CHCH₂O-, especially in the trans form, -(CH₂)₅ -, -(CH₂)₆-, -(CH₂) ₃O-, -(CH₂)₅O-, -(CH₂)₆O- or 1,4-phenylene and/or
c) R³ is H, Br, Cl, F or CH₃ and/or
d) Q is propyl, pentyl, isohexyl, 4-methyl-3-pentenyl or 2,6-dimethyl-5-heptenyl or
e) Q is a group Q' in which R is H, Br, Cl, F, I, CF₃, CN or NO₂ and/or R⁴ is H, Br, Cl, F or CH₃, and/or R⁵ is H or F.

4. Use in accordance with claim 1 or 2, whereby
a) L is C₅₋₁₁-alkylene or C₅₋₁₁-alkyleneoxy, especially -(CH₂)₆- or -(CH₂)₅O-; C₃₋₁₁-alkenyleneoxy, especially -CH=CHCH₂O-, or 1,4-phenylene and/or
b) R³ is H or halogen and/or
c) Q is C₂₋₁₀-alkenyl, especially 4-methyl-3-pentenyl; or a group Q' in which R is CN, NO₂ or halogen, especially Br, Cl or F, and R⁴ is H or Cl.

5. Use in accordance with claim 4, whereby
a) R¹ is methyl and R² is methyl or allyl and/or
b) L is -(CH₂)₅O-, -CH=CHCH₂O- or 1,4-phenylene and/or
c) R³ is H or F and/or
d) Q is 4-methyl-3-pentenyl or a group Q' in which R is Br, Cl, CN or NO₂, R⁴ is H or Cl and R⁵ is H.

6. Use in accordance with claim 1 or 2, whereby the compound is selected from the following group:
trans-4-[[4-(Allylmethylamino)-2-butenyl]oxy]-4'-bromobenzophenone,
trans-4-[[4-(allylmethylamino)-2-butenyl]oxy]-4'-nitrobenzophenone,
p-[[4'-[(allylmethylamino)methyl]-4-biphenylyl]carbonyl]benzonitrile,
2-chloro-4-nitrophenyl 4'-[(dimethylamino)methyl]-4-biphenylyl ketone,
trans-4-[[4-(allylmethylamino)-2-butenyl]oxy] -2',4'-dichlorobenzophenone.

7. Use in accordance with claim 1, whereby the compound is selected from the following group:
[4-[6-(Allyl-methyl-amino)-hexyloxy]-phenyl]-(4-bromo-phenyl)-methanone,
[4-[6-(allyl-methyl-amino)-hexyloxy]-3-fluoro-phenyl]-(4-bromo-phenyl)-methanone,
[4-[6-(allyl-methyl-amino)-hexyloxy] -2-fluoro-phenyl]-(4-bromo-phenyl)-methanone,
(E)-[4-[4-(allyl-methyl-amino)-but-2-enyloxy]-3-fluoro-phenyl]-(4-bromo-phenyl)-methanone,
(E)-1-[4-[4-(allyl-methyl-amino)-but-2-enyloxy]-phenyl]-5-methyl-hex-4-en-1-one,
(E)-[4-[4-(allyl-methyl-amino)-but-2-enyloxy]-2-fluoro-phenyl] -(4-bromo-phenyl)-methanone.

8. The compounds of formula I in accordance with claim 1 or 2 selected from the following group:
4-[[6-(Allylmethylamino)hexyl]oxy]-3-chlorobenzophenone,
4-[[6-(allylmethylamino)hexyl]oxy]-3,4'-dibromobenzophenone,
4-[[4-(allylmethylamino)-2-butenyl]oxy]-3,4'-dibromobenzophenone,
3-chloro-4'-iodo-4-[[6-(allylmethylamino)hexyl]oxy]benzophenone,
4'-bromo-3-chloro-4-[[6-(allylmethylamino)hexyl]oxy]benzophenone,
2,4-[[(4-dimethylamino)-2-butenyl]oxy]-3,4'-dibromobenzophenone,
4-[[4-(dimethylamino)-2-butenyl]oxy]-3-chlorobenzophenone,
4'-bromo-3-chloro-4-[[6-(dimethylamino)hexyl]oxy]benzophenone,
3,4-dichlorophenyl 4'-[(dimethylamino)methyl]-4-biphenylyl ketone,
4'-[(allylmethylamino)methyl]-4-biphenylyl 3,4-dichlorophenyl ketone,
(RS)-4'-(dimethylaminomethyl)-4-biphenyl 2,6-dimethyl-5-heptenyl ketone,
p-bromphenyl 2-chloro-4'-[(dimethylamino)methyl]-4-biphenylyl ketone,
4'-[(dimethylamino)methyl]-4-biphenylyl propyl ketone.

9. The compounds of formula I in accordance with claim 1 selected from the following group:
[4-[6-(Allyl-methyl-amino)-hexyloxy]-phenyl] -(4-bromo-phenyl)-methanone,
[4-[4-(allyl-methyl-amino)-butoxy]-phenyl]-(4-bromo-phenyl)-methanone,
[4-[6-(allyl-methyl-amino)-hexyloxy]-3-fluoro-phenyl]-(4-bromo-phenyl)-methanone,
[4-[6-(allyl-methyl-amino)-hexyloxy]-2-fluoro-phenyl]-(4-bromo-phenyl)-methanone,
(E)-[4-[4-(allyl-methyl-amino)-but-2-enyloxy]-phenyl]-(4-trifluoromethyl-phenyl)-methanone,
(E)-[4-[4-(allyl-methyl-amino)-but-2-enyloxy]-3-fluoro-phenyl]-(4-bromo-phenyl)-methanone,
(E)-1-[4-[4-(allyl-methyl-amino)-but-2-enyloxy]-phenyl]-5-methyl-hexan-1-one,
(E)-[4-[4-(allyl-methyl-amino)-but-2-enyloxy]-phenyl]-(4-iodo-phenyl)-methanone,
(E)-1-[4-(allyl-methyl-amino)-but-2-enyloxy]-3-fluoro-phenyl]-5-methyl-hexan-1-one,
(E)-[4-[4-(allyl-methyl-amino)-but-2-enyloxy]-3-fluoro-benzoyl]-benzonitrile,
(E)-4-[4-[4-(allyl-methyl-amino)-but-2-enyloxy]-benzoyl]-benzonitrile,
(E)-[4-[4-(allyl-methyl-amino)-but-2-enyloxy]-3-fluoro-phenyl]-(2,6-difluoro-phenyl)-methanone,
(E)-1-[4-[4-(allyl-methyl-amino)-but-2-enyloxy]-phenyl]-5-methyl-hex-4-en-1-one,
(E)-[4-[4-(allyl-methyl-amino)-but-2-enyloxy]-2-fluoro-phenyl]-(4-bromo-phenyl)-methanone,
(E)-[4-[4-(allyl-methyl-amino)-but-2-enyloxyl-phenyl]-(4-fluoro-phenyl)-methanone,
(E)-1-[4-[4-(allyl-methyl-amino)-but-2-enyloxy]-3-fluoro-phenyl]-6-methyl-hept-5-en-2-one,
(E)-2-[4-[4-(allyl-methyl-amino)-but-2-enyloxy]-3-fluoro-phenyl]-1-(4-bromo-phenyl)-ethanone,
(E)-2-[4-[4-(allyl-methyl-amino)-but-2-enyloxy)-phenyl]-1-(4-bromo-phenyl)-ethanone,
(E)-(4-bromo-phenyl)-[4-[4-(ethyl-methyl-amino)-but-2-enyloxy]-phenyl]-methanone,
4'-[(allylmethylamino)methyl]-2-chloro-4-biphenyly p-bromophenyl ketone.

## Revendications

1. Utilisation des composés de formule 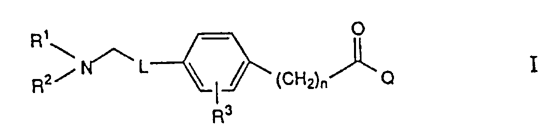 dans laquelle
l'un des radicaux R¹ et R² est un groupe alkyle en C₁₋₇ et l'autre est un groupe alkyle en C₁₋₇ ou (alcényle en C₂₋₆)méthyle,
L est un groupe alkylène en C₁₋₁₁ ou alcénylène en C₂₋₁₁ éventuellement lié au groupe phényle par l'intermédiaire d'un atome d'oxygène, ou encore L est le groupe 1,4-phénylène,
n vaut 0, ou encore, si L contient un atome d'oxygène, n vaut 0 ou 1,
Q est un groupe alkyle en C₁₋₇, alcényle en C₂₋₁₀, ou un groupe de formule Q' : 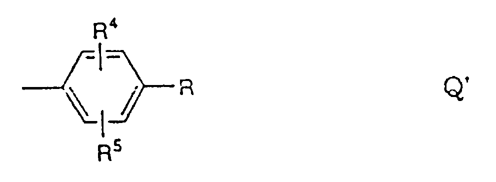
R est H, un halogène, CF₃, CN ou NO₂,
R³ et R⁴ sont H ou des groupes alkyle en C₁₋₄ ou halogéno, et
R⁵ est H ou encore, si R est H, H ou un atome d'halogène,
et leurs sels d'addition avec un acide acceptables d'un point de vue pharmaceutique, pour la préparation de médicaments hypocholestérolémiques.

2. Utilisation selon la revendication 1, dans laquelle n vaut 0 et R⁵ est H.

3. Utilisation selon la revendication 1 ou 2, dans laquelle
a) R¹ est le groupe méthyle et R⁵ est le groupe méthyle, éthyle, propyle ou allyle, et/ou
b) L est le groupe -CH=CHCH₂O-, en particulier sous forme trans, -(CH₂)₅-, -(CH₂)₆-, -(CH₂)₃O-, -(CH₂)₅O-, -(CH₂)₆O- ou le groupe 1,4-phénylène, et/ou
c) R³ est H, Br, Cl, F ou CH₃, et/ou
d) Q est le groupe propyle, pentyle, isohexyle, 4-méthyl-3-pentényle ou 2,6-diméthyl-5-heptényle, ou
e) Q est un groupe Q' dans lequel R est H, Br, Cl, F, I, CF₃, CN ou NO₂, et/ou R⁴ est H, Br, Cl, F ou CH₃ et/ou R⁵ est H ou F.

4. Utilisation selon la revendication 1 ou 2, dans laquelle
a) L est un groupe alkylène en C₅₋₁₁ ou alkylénoxy en C₅₋₁₁, en particulier -(CH₂)₆- ou -(CH₂)₅O- ; un groupe alcénylénoxy en C₃₋₁₁, en particulier - CH=CHCH₂O-, ou le groupe 1,4-phénylène, et/ou
b) R³ est H ou un halogène, et/ou
c) Q est un groupe alcényle en C₂₋₁₀, en particulier le groupe 4-méthyl-3-pentényle ; ou un groupe Q' dans lequel R est CN, NO₂ ou un halogène, en particulier Br, Cl ou F, et R⁴ est H ou Cl.

5. Utilisation selon la revendication 4, dans laquelle
a) R¹ est le groupe méthyle et R² est le groupe méthyle ou allyle, et/ou
b) L est -(CH₂)₅O-, -CH=CHCH₂O- ou le groupe 1,4-phénylène, et/ou
c) R³ est H ou F, et/ou
d) Q est le groupe 4-méthyl-3-pentényle ou un groupe Q' dans lequel R est Br, Cl, CN ou NO₂, R⁴ est H ou Cl et R⁵ est H.

6. Utilisation selon la revendication 1 ou 2, dans laquelle les composés sont choisis dans le groupe suivant :
trans-4-[[4-(allylméthylamino)-2-butényl]-oxy]-4'-bromobenzophénone,
trans-4-[[4-(allylméthylamino)-2-butényl]-oxy]-4'-nitrobenzophénone,
p-[[4'-[(allylméthylamino)-méthyl]-4-biphénylyl]-carbonyl]-benzonitrile,
2-chloro-4-nitrophényl-4'-[(diméthylamino)-méthyl]-4-biphénylylcétone,
trans-4-[[4-(allylméthylamino)-2-butényl]-oxy]-2'-4'-dichlorobenzophénone.

7. Utilisation selon la revendication 1, dans laquelle les composés sont choisis dans le groupe suivant :
[4-[6-(allylméthylamino)-hexyloxy]-phényl]-(4-bromophényl)-méthanone,
[4-[6-(allylméthylamino)-hexyloxy]-3-fluorophényl]-(4-bromophényl)-méthanone,
[4-[6-(allylméthylamino)-hexyloxy]-2-fluorophényl]-(4-bromophényl)-méthanone,
(E)-[4-[4-(allylméthylamino)-but-2-ényloxy]-3-fluorophényl]-(4-bromo-phényl)-méthanone,
(E)-1-[4-[4-(allylméthylamino)-but-2-ényloxy]-phényl]-5-méthylhex-4-ène-1-one,
(E)-[4-[4-(allylméthylamino)-but-2-ényloxy]-2-fluorophényl]-(4-bromo-phényl)-méthanone.

8. Composés de formule I selon la revendication 1 ou 2, choisis dans le groupe suivant :
4-[[6-(allylméthylamino)-hexyl]-oxy]-3-chlorobenzophénone,
4-[[6-(allylméthylamino)-hexyl]-oxy]-3,4'-dibromobenzophénone,
4-[[4-(allylméthylamino)-2-butényl]-oxy]-3,4'-dibromobenzophénone,
3-chloro-4'-iodo-4-[[6-(allylméthylamino)-hexyl]-oxy]-benzophénone,
4'-bromo-3-chloro-4-[[6-(allylméthylamino)-hexyl]-oxy]-benzophénone,
2,4-[[(4-diméthylamino)-2-butényl]-oxy]-3,4'-dibromobenzophénone,
4-[[4-(diméthylamino)-2-butényl]-oxy]-3-chlorobenzophénone,
4'-bromo-3-chloro-4-[[6-(diméthylamino)-hexyl]-oxy]-benzophénone,
3,4-dichlorophényl-4'-[(diméthylamino)-méthyl]-4-biphénylylcétone,
4'-[(allylméthylamino)-méthyl]-4-biphénylyl-3,4-dichlorophénylcétone,
(RS)-4'-(diméthylaminométhyl)-4-biphényle-2,6-diméthyl-5-hepténylcétone,
p-bromophényl-2-chloro-4'-[(diméthylamino)-méthyl]-4-biphénylylcétone,
4'-[(diméthylamino)-méthyl]-4-biphénylylpropylcétone.

9. Composés de formule I selon la revendication 1, choisis dans le groupe suivant :
[4-[6-(allylméthylamino)-hexyloxy]-phényl]-(4-bromophényl)-méthanone,
[4-[4-(allylméthylamino)-butoxy[-phényl]-(4-bromophényl)-méthanone,
[4-[6-(allylméthylamino)-hexyloxy]-3-fluorophényl]-(4-bromophényl)-méthanone,
[4-[6-(allylméthylamino)-hexyloxy]-2-fluorophényl]-(4-bromophényl)-méthanone,
(E)-[4-[4-(allylméthylamino)-but-2-ényloxy]-phényl]-(4-trifluorométhylphényl)-méthanone,
(E)-[4-[4-(allylméthylamino)-but-2-ényloxy]-3-fluorophényl]-(4-bromo-phényl)-méthanone,
(E)-1-[4-[4-(allylméthylamino)-but-2-ényloxy]-phényl]-5-méthylhexane-1-one,
(E)-[4-[4-(allylméthylamino)-but-2-ényloxy]-phényl]-(4-iodophényl)-méthanone,
(E)-1-[4-(allylméthylamino)-but-2-ényloxy]-3-fluorophényl]-5-méthyl-hexane-1-one,
(E)-[4-[4-(allylméthylamino)-but-2-ényloxy]-3-fluorobenzoyl]-benzonitrile,
(E)-4-[4-[4-(allylméthylamino)-but-2-ényloxy]-benzoyl]-benzonitrile,
(E)-[4-[4-(allylméthylamino)-but-2-ényloxy]-3-fluorophényl]-(2,6-difluorophényl)-méthanone,
(E)-1-[4-[4-(allylméthylamino)-but-2-ényloxy]-phényl]-5-méthylhex-4-ène-1-one,
(E)-[4-[4-(allylméthylamino)-but-2-ényloxy]-2-fluorophényl]-(4-bromophényl)-méthanone,
(E)-[4-[4-(allylméthylamino)-but-2-ényloxy]-phényl]-(4-fluorophényl)-méthanone,
(E)-1-[4-[4-(allylméthylamino)-but-2-ényloxy]-3-fluorophényl]-6-méthyl-hept-5-ène-2-one,
(E)-2-[4-[4-(allylméthylamino)-but-2-ényloxy]-3-fluorophényl]-1-(4-bromophényl)-éthanone,
(E)-2-[4-[4-(allylméthylamino)-but-2-ényloxy]-phényl]-1-(4-bromophényl)-éthanone,
(E)-(4-bromophényl)-[4-[4-(éthylméthylamino)-but-2-ényloxy]-phényl]-méthanone,
4'-[(allylméthylamino)méthyl]-2-chloro-4-biphénylyl-p-bromophénylcétone
